# EUROPEAN PATENT SPECIFICATION

(11) **EP 2 863 899 B1**
(45) Date of publication and mention of the grant of the patent: **02.11.2016**
(21) Application number: 13739368.2
(22) Date of filing: 21.06.2013
(51) Int. Cl.: A61K 9/20, A61K 31/567

(54) **FORMULATIONS FOR THE PREPARATION OF IMMEDIATE-RELEASE TABLETS FOR ORAL USE CONTAINING LOW-DOSE MIFEPRISTONE FOR THE TREATMENT OF ENDOMETRIOSIS, TABLETS THUS OBTAINED AND THEIR PREPARATION PROCESS**
FORMULIERUNGEN ZUR HERSTELLUNG VON SCHNELLAUFLÖSENDEN ORALEN TABLETTEN MIT NIEDRIGDOSIERTEM MIFEPRISTON ZUR BEHANDLUNG VON ENDOMETRIOSE, DAMIT HERGESTELLTE TABLETTEN UND DEREN HERSTELLUNGSVERFAHREN
FORMULATIONS DE PRÉPARATION DE COMPRIMÉS À LIBÉRATION IMMÉDIATE POUR ADMINISTRATION ORALE DE MIFEPRISTONE FAIBLEMENT DOSÉ AFIN DE TRAITER L'ENDOMÉTRIOSE, COMPRIMÉS AINSI OBTENUS ET LEUR PROCÉDÉ DE PRÉPARATION

(30) Priority: 21.06.2012 IT FI20120128
(43) Date of publication of application: 29.04.2015
(73) Proprietor: Vapharma International S.p.A., 47864 Pennabilli (IT)
(72) Inventor: VALDUCCI, Roberto, 47039 Savignano Sul Rubicone (IT); AVANESSIAN, Serozh, 47827 Villa Verucchio (IT)
(74) Representative: Brighenti, Livio
(86) International application number: PCT/EP2013/063011
(87) International publication number: WO 2013/190098

(56) References cited:
- EP-A1- 1 987 814
- EP-A1- 2 210 585
- CN-A- 101 455 671

## Description

### Field of the invention

The present invention relates to the field of pharmaceutical compositions for the treatment of endometriosis.

### Background of the invention

As is known, endometriosis is a chronic and complex disease that is often painful to the extent of being disabling, originating from an excessive presence of the tissue that covers the inner wall of the uterus, that is the endometrium, or of other organs such as ovaries, tubes, peritoneum, vagina, intestine.

It is estimated that about 10% of women in Europe are affected by endometriosis and that 30% to 40% of cases of female infertility is due to endometriosis; in Italy there are at least 3 million women with a confirmed diagnosis of endometriosis. To date there are no known therapies capable of permanently resolving endometriosis to date have yet to be found.

It is also known that Mifepristone, also known as RU486 with chemical formula 11β- [4-(N,N-Dimethylamino)-phenyl-17β-hydroxy-17α-(1-propynyl)-estra-4,9-dien-3-one, is a selective progesterone antagonist at receptor level without progestogenic, oestrogenic, androgenic and anti-oestrogenic activity. This molecule's ability to bind with the progesterone receptor at endometrial level is 5 times higher with respect to that of the progesterone itself. It can be quickly absorbed following oral administration with the appearance of a peak at blood level after just 1 and half hours. It has a long half-life time of around 20 hours (much longer than that of many glucocorticoid agonists), which makes its in vivo clearance very slow. It too is naturally subject to the first passage effect and the blood level of its metabolites after 1-2 hours from oral administration is greater with respect to the original compound. The use of high-dose mifepristone has proved effective as emergency post-coital contraceptive treatment for hospital use in combination with misoprostol by oral route or prostaglandin E₁ by vaginal route. There are prior art studies that have demonstrated the efficacy of mifepristone for the treatment of other diseases such as leimyoma, myoma, uterine fibrosis, endometriosis, adenomyosis and related disorders.

Eisinger et al (2003) have demonstrated that the daily oral administration of a low-dose of Mifepristone (5 mg) for a period of 6 months has effects comparable with those of higher doses of Mifepristone in the treatment of uterine fibrosis with reduction of the related side effects.

EP 2 210 585 discloses 3 different mifepristone compositions formulated as tablets for the treatment of uterine fibroids, the compositions are given in % (w/w). The first composition consists of: mifepristone 1.02 %; MCC 62.98%; polyvinylpyrrolidone 10%; Mg stearate 2%; HPMC 14% and crospovidone 10%. The second composition consists of: mifepristone 1.02 %; MCC 46.98%; polyvinylpyrrolidone 10%; Mg stearate 2%; HPMC 14%; lactose 16% and croscarmellose Na 10%. The third composition consists of: mifepristone 1.02 %; MCC 54.98%; polyvinylpyrrolidone 10%; Mg stearate 2%; HPMC 12%; lactose 5% and croscarmellose Na 15%.

It should also be borne in mind that mifepristone, being equipped with a high activity, requires special conditions for the safe processing thereof, which must take place in suitable premises and with equipment capable of allowing processing in containment conditions.

It is thus evident, from what has been said above, how useful it would be to have low-dose mifepristone formulations in the form of immediate-release tablets for oral use for the treatment of endometriosis.

### Summary of the invention

The present invention relates to formulations described in the claim.

### Detailed description of the invention

The present invention allows the above-mentioned problems to be resolved thanks to a formulation containing Mifepristone for the treatment of endometriosis and to a production process that does not require special procedures, allows the problems due to the handling of the mifepristone to be reduced and thus allows immediate-release tablets for oral use containing a low dose of the active ingredient, to be made available.

A tablet according to the invention, has the following composition (expressed by weight on the total weight):

| | |
|---|---|
| Mifepristone | 6 % |
| Microcrystalline Cellulose (with a water content 1-10%w/w) | 85.7 % |
| Polyvinylpyrrolidone | 4 % |
| Croscarmellose Sodium | 3 % |
| Magnesium Stearate | 1 % |
| Anhydrous Colloidal Silica | 0.3 % |

According to a disclosed embodiment, the tablets are produced by the formation of a premix wherein the active ingredient is mixed with an equal amount of microcrystalline cellulose and is sieved.

In the meantime, the remaining microcrystalline cellulose, binder and disintegrant are sieved and mixed together. To this mixture is added and mixed the premix containing the active ingredient. Lastly, the lubricants and glidants are added and this mixture is compressed.

Using the above-described components and process, it is possible to obtain immediate-release, low-dose Mifepristone tablets for oral administration that guarantee the uniformity of content requirements with excellent uniformity of content. It is thus possible to easily obtain, and with fewer risks, low-dose mifepristone tablets that guarantee therapeutic efficacy in the treatment of endometriosis with fewer side effects.

### Example

Formulation of low-dose, immediate-release mifepristone tablets with excellent uniformity of content.

For a batch of 1,000,000 tablets 5 kg of raw material 71.417 kg of microcrystalline cellulose, 3.333 kg of polyvinylpyrrolidone, 2.5 kg of croscarmellose sodium, 0.833 kg of magnesium stearate and 0.250 kg of anhydrous colloidal silica were used.
a) Preparation of the Premix The mifepristone and the microcrystalline cellulose are mixed together and the mixture is sieved.
b) Mixing of the excipients The remaining microcrystalline cellulose, polyvinyl pyrrolidone and croscarmellose sodium are mixed together.
c) Mixing of the ingredients with the active premix The excipient mixture is added to the premix containing the active ingredient and mixed in BIN for 10 minutes at 8 rpm.
d) Addition of lubricants and glidants The magnesium stearate and microcrystalline silica are sieved and added to the BIN to be mixed with the remaining components.
e) Compression of the mixture. The mixture is compressed to obtain tablets having a 6mm diameter and average weight of 80 mg with active ingredient content equal to 5 mg/TBL.

## Claims

1. Immediate-release tablets for oral administration consisting of:
Mifepristone 6% w/w;
Microcristalline Cellulose (with a water content 1-10% w/w) 85.7% w/w;
Polyvinylpyrrolidone 4% w/w;
Croscarmellose Sodium 3% w/w;
Magnesium Stearate 1% w/w;
Anhydrous Colloidal Silica 0.3 % w/w;
wherein the tablets are for use in a method of treatment of endometriosis.

## Patentansprüche

1. Tabletten mit unmittelbarer Freisetzung zur oralen Verabreichung, zusammengesetzt aus:
Mifepriston 6 Gew.-%
Mikrokristalline Zellulose (mit einem Wassergehalt von 1 - 10 Gew.-%) 85,7 Gew.-% Polyvinylpyrrolidon 4 Gew.-%
Croscarmellose-Natrium 3 Gew.-%
Magnesium-Stearat 1 Gew.-%
Anhydrische Kolloid-Kieselerde 0,3 Gew.-%
wobei die Tabletten zur Behandlung von Endometriose verwendet werden.

## Revendications

1. Comprimés à libération immédiate pour une administration orale constitués de :
mifépristone 6 % p/p ;
cellulose microcristalline (avec une teneur en eau de 1 à 10 % p/p) 85,7 % p/p ;
polyvinylpyrrolidone 4 % p/p ;
croscarmellose sodique 3 % p/p ;
stéarate de magnésium 1 % p/p ;
silice colloïdale anhydre 0,3 % p/p ;
les comprimés étant destinés à une utilisation dans un procédé de traitement de l'endométriose.
